# EUROPEAN PATENT APPLICATION

(11) **EP 4 736 910 A1**
(43) Date of publication of application: **06.05.2026**
(21) Application number: 23962065.1
(22) Date of filing: 27.12.2023
(51) Int. Cl.: A61M 5/20

(54) **NOVEL LUBAN LOCK STRUCTURE AND APPLICATION METHOD THEREFOR IN SYRINGE**

(30) Priority: 20.12.2023 CN 202311758527
(71) Applicant: Eases Medical Technology (Suzhou) Co., Ltd, Suzhou, Jiangsu 215000 (CN)
(72) Inventor: LIU, Kai, Suzhou, Jiangsu 215000 (CN)
(74) Representative: Bayramoglu et al.
(86) International application number: PCT/CN2023/142415
(87) International publication number: WO 2025/129743

(57) **Abstract**

The present invention discloses a novel Luban lock structure, including: a booster rod, a power source, a fixing block, a guide pin, a sound-emitting block, and a slider. The slider is slidably coupled to an outer side of the sound-emitting block. The booster rod is engaged with an inner side of the sound-emitting block via a first boss. The slider is provided with a stop plate configured to limit a position of the first boss. The fixing block is snap-fitted between a top end of the sound-emitting block and a top end of the slider. The guide pin is connected to an inner bottom end of the sound-emitting block. The power source is sleeved over the guide pin, with two ends of the power source respectively connected to an end of the guide pin and an end of a cavity of the booster rod. When the Luban lock structure of the present invention is applied to an autoinjector, the booster rod is prevented from moving forward when the first boss of the booster rod is engaged with a guiding inclined surface of the sound-emitting block while the stop plate of the slider extends into a channel of the sound-emitting block, thereby preventing accidental triggering of a power unit of the autoinjector. The booster rod is free of grooves, which increases structural strength, reduces fracture risks during prolonged storage, extends the service life of the autoinjector, lowers manufacturing precision requirements, and reduces production costs.

## Description

### TECHNICAL FIELD

The present invention relates to the technical field of medical devices, and more specifically, the present invention relates to a novel Luban lock structure and a method for applying the same to an autoinjector.

### BACKGROUND TECHNOLOGY

A disposable injection pen is a pen-type injector pre-filled with medicament, also known as a pre-filled injection pen. Currently, the working principles of the power mechanism of injection pens on the market are all the same, that is, after a spring (power source) is pre-compressed, the spring is released during use, and then a plunger rod is driven forward to perform injection. Currently, the injection pens on the market all adopt a clamping-type structure.

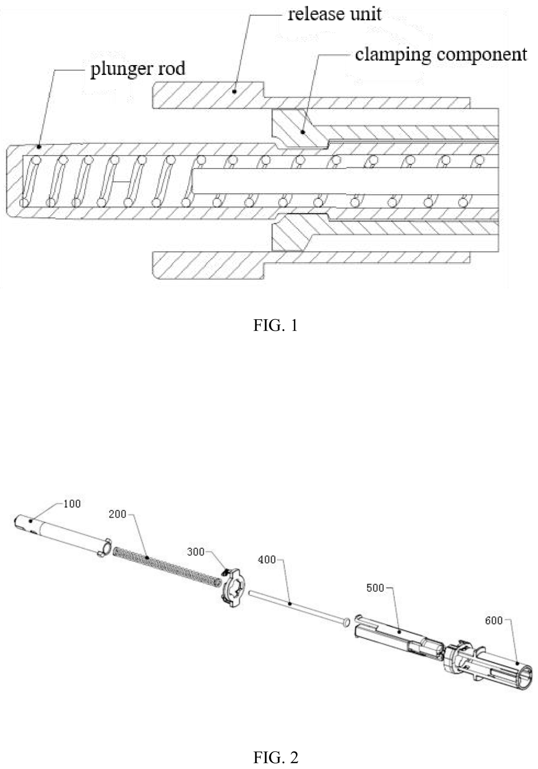

FIG. 1 provides a structural view of a power mechanism of an injection pen currently used on the market, with its working principle as follows: a spring is installed into a plunger rod for pre-compression, and then clamped in an annular groove of the plunger rod by a clamping component, and then a release unit is installed so that the clamping component cannot deform, thereby ensuring that the plunger rod remains fixed and stationary in a state where the spring is compressed. During release, the release unit is pressed downward; when the release unit moves past the clamping component, under the action of the spring, two hooks of the clamping component deform outward, and the plunger rod is then pushed forward to realize the function of injection. This form has the following problems: (1) due to space limitations, the depth of the annular groove on the plunger rod is only 0.5 mm, and the space for the clamping component is relatively small, which requires high dimensional precision of the product, thereby increasing the difficulty of manufacturing and also increasing the cost of parts; (2) due to the high precision requirements and limited space for the clamping component, accidental triggering may occur during actual use; (3) since the annular groove is cut on the plunger rod, the plunger rod may break during long-term storage.

Therefore, it is necessary to propose a novel Luban lock structure and a method for applying the same to an autoinjector, so as to at least partially address the problems existing in the prior art.

### CONTENT OF THE INVENTION

In the "Summary" section of the present invention, a series of simplified concepts are introduced, which will be further described in the "Detailed Description of The Embodiments" section. The "Summary" section of the present invention is not intended to limit the key and necessary technical features of the claimed technical solution, nor is it intended to determine the scope of protection of the claimed technical solution.

To at least partially solve the above problems, the present invention provides a novel Luban lock structure, including:
a booster rod, a power source, a fixing block, a guide pin, a sound-emitting block and a slider, wherein the slider is slidably coupled to an outer side of the sound-emitting block; the booster rod is engaged with an inner side of the sound-emitting block via a first boss; the slider is provided with a stop plate configured to limit a position of the first boss; the fixing block is snap-fitted between a top end of the sound-emitting block and a top end of the slider; the guide pin is connected to an inner bottom end of the sound-emitting block; the power source is sleeved over the guide pin; and two ends of the power source are respectively connected to an end of the guide pin and an end of a cavity of the booster rod.

Preferably, the sound-emitting block is configured as a hollow structure with an open top end, including:
limiting slots, wherein two limiting slots are symmetrically formed at a lower part of the sound-emitting block and extend through a side wall thereof;
channels, wherein two channels are symmetrically connected to an outer side of the sound-emitting block and extend along a length direction of the sound-emitting block; the channels are in communication with the limiting slots, and a width of the limiting slots is greater than a width of the channels; a guiding inclined surface is formed on a lower end surface where each channel is connected to the corresponding limiting slot; and a height of the guiding inclined surface gradually increases from an inner side to an outer side of the channel;
second cantilevers, wherein two second cantilevers are symmetrically connected to an upper part of the sound-emitting block; the second cantilevers and the channels are arranged in a staggered manner, with a gap provided between them; and
hooks, wherein each hook is connected to a top end of the second cantilever and folded outward; and a bottom surface of each hook is provided with a hook inclined surface.

Preferably, the booster rod is configured as a hollow structure with an open bottom end, including:
first bosses, wherein two first bosses are symmetrically provided at both sides of a bottom end of the booster rod; an upper surface of each first boss is provided with an upper inclined surface configured to be engaged with the guiding inclined surface of the sound-emitting block; and a lower surface of each first boss is provided with a lower inclined surface; and
positioning flats, wherein two positioning flats are symmetrically connected to a top end of the booster rod.

Preferably, the power source is configured as an elastic reset element.

Preferably, the fixing block includes:
a first through hole extending through a center of the fixing block;
second bosses, wherein two second bosses are symmetrically connected to both sides of the fixing block;
first cantilevers, wherein each first cantilever is connected to a top end of the second boss; and a top end of each first cantilever is bent inward;
first side recesses, wherein two first side recesses are symmetrically disposed in the first through hole and correspond to positions of the first cantilevers; and a recess inclined surface is provided on a top surface of each first side recess, which is configured to be in contact with the hook inclined surface; and
second side recesses, wherein two second side recesses are symmetrically disposed in the first through hole and arranged in a staggered manner with the first side recesses; and a distance from each second side recess to the center of the fixing block is less than a distance from an outer side surface of the channel to a center of the sound-emitting block.

Preferably, the slider is configured as a hollow structure provided with two open ends, including:
a fixing seat, wherein the fixing seat is connected to a top end of the slider and having a second through hole formed through a center of the fixing seat; the fixing block is mounted within the fixing seat; receiving slots are symmetrically formed on both sides of the fixing seat; and the second boss is mounted therein;
third side recesses, wherein two third side recesses are symmetrically formed on both sides of the second through hole; the third side recesses are arranged in a staggered manner with the receiving slots; and a distance from each third side recess to a center of the slider is greater than the distance from the outer side surface of the channel to the center of the sound-emitting block; and
limiting holes, wherein two limiting holes are symmetrically formed in a side wall of the slider; and positions of the limiting holes correspond to those of the receiving slots.

Preferably, the slider further includes:
elongated slots, wherein two elongated slots are symmetrically formed in both sides of the slider and extend along a length direction of the slider; the elongated slots are arranged in a staggered manner with the limiting holes and communicate with the third side recesses; and the channel is slidable into the corresponding elongated slot along the third side recesses; and
stop plates, wherein each stop plate is connected to a bottom end of the elongated slot and extends along the length direction of the slider; and the stop plate is mounted in the channel;
support arms, wherein two support arms are symmetrically connected to an outer wall of the slider; and the support arms are disposed below the limiting holes.

Preferably, the specific assembly process of the novel Luban lock structure includes the following steps:
step S100, inserting the sound-emitting block into the slider;
step S200, fitting the fixing block over the sound-emitting block;
step S300, inserting the guide pin into the sound-emitting block;
step S400, fitting the power source over the guide pin via a center thereof;
step S500, fitting the booster rod over the power source;
step S600, pressing the booster rod to pass through the sound-emitting block, then rotating the booster rod to engage the first bosses of the booster rod with the guiding inclined surfaces of the sound-emitting block; and
step S700, pushing the slider upward to insert the two stop plates of the slider into the corresponding channels of the sound-emitting block, thereby completing the assembly.

A method for applying a novel Luban lock structure to an autoinjector, employing the novel Luban lock structure, wherein the novel Luban lock structure is installed within the autoinjector to form a drug delivery booster device module, and the drug delivery booster device module includes:
a first housing, wherein the first housing is sleeved over an outer side of the novel Luban lock structure and having one end provided with an opening; the booster rod extends out of the first housing; a side wall of the first housing is symmetrically provided with snap slots; an edge of the second boss of the fixing block is snap-fitted within the snap slot; and an end of the sound-emitting block is spaced apart from an end of an inner wall of the first housing; and
a support power source, wherein the support power source is sleeved over an outer side of the slider; and two ends of the support power source are respectively connected to the end of the inner wall of the first housing and the support arm.

Preferably, the autoinjector further includes a drug delivery protection device module, and the drug delivery protection device module includes:
a second housing having one end connected to an open end of the first housing, and the other end provided with an opening;
a drug chamber, wherein the drug chamber is installed at a center of the second housing; a drug liquid is accommodated within the drug chamber and is blocked by a rubber plug; the first cantilever is snap-fitted with one end of the drug chamber; the booster rod extends into the drug chamber and is adjacent to the rubber plug; and the other end of the drug chamber is connected to a needle;
a protective cover detachably connected to an open end of the second housing and fitted over an outer side of the needle; and
a telescopic shroud, wherein the telescopic shroud is telescopically connected to the open end of the second housing; and the telescopic shroud is connected to the slider via a structural member.

Compared with the prior art, the present invention includes at least the following beneficial effects:
Regarding the novel Luban lock structure provided by the present invention, during use, the slider is moved in a positive direction of the Z-axis until the stop plate on the slider completely exits the channel of the sound-emitting block, at which time the first boss of the booster rod loses limitation; under the action of the power source and the guiding inclined surface of the sound-emitting block, the booster rod rotates along the guiding inclined surface of the sound-emitting block, and when the first boss of the booster rod rotate into the channel of the sound-emitting block, it starts to move in a negative direction of the Z-axis; when the booster rod is completely moved out of the sound-emitting block, the power source still maintains a certain force, which will push the sound-emitting block to move in the positive direction of the Z-axis and collide with subsequent components, thereby forming a sound. When the slider resets (moves in the negative direction of the Z-axis), the hook inclined surface on the sound-emitting block and the recess inclined surface slide relative to each other, the hook is compressed into the slider, and as it slides, the hook snaps into engagement with the limiting holes on the side surface of the slider, thereby forming protection.

When the novel Luban lock structure provided by the present invention is applied to an autoinjector, with the first boss of the booster rod engaged with the guiding inclined surface of the sound-emitting block, and simultaneously the stop plate of the slider inserted into the channel of the sound-emitting block, the booster rod is prevented from moving forward, which solves the problem of accidental triggering of the power unit of the current autoinjectors; the booster rod is free of grooves, which enhances its strength, reduces the risk of fracture during long-term storage, extends the service life of the autoinjector, lowers the requirements for structural manufacturing precision, and reduces production costs.

Regarding the novel Luban lock structure and the method for applying the same to an autoinjector according to the present invention, other advantages, objectives, and features of the present invention will be partially embodied through the following description, and some will be understood by those skilled in the art through the research and practice for the present invention.

### DESCRIPTION OF THE DRAWINGS

The drawings are provided for further understanding of the present invention, and constitute a part of the specification. The drawings, together with the embodiments of the present invention, are intended to explain the present invention, rather than to limit the present invention. In the drawings:
FIG. 1 is a structural view of a power mechanism of an injection pen used on the market;
FIG. 2 is a schematic exploded structural view of a novel Luban lock structure according to the present invention;
FIG. 3 is a schematic structural view of a booster rod and a partially enlarged schematic structural view at A in a novel Luban lock structure according to the present invention;
FIG. 4 is a schematic structural view of a fixing block in a novel Luban lock structure according to the present invention;
FIG. 5 is a first schematic structural view of a sound-emitting block in a novel Luban lock structure according to the present invention;
FIG. 6 is a second schematic structural view of a sound-emitting block in a novel Luban lock structure according to the present invention;
FIG. 7 is a schematic structural view of a slider in a novel Luban lock structure according to the present invention;
FIG. 8 is a schematic view of an installation state of step S100 during an assembly process of a novel Luban lock structure according to the present invention;
FIG. 9 is a schematic view of an installation state of step S200 during an assembly process of a novel Luban lock structure according to the present invention;
FIG. 10 is a schematic view of an installation state of step S300 during an assembly process of a novel Luban lock structure according to the present invention;
FIG. 11 is a schematic view of an installation state of step S400 during an assembly process of a novel Luban lock structure according to the present invention;
FIG. 12 is a schematic view of an installation state of step S500 during an assembly process of a novel Luban lock structure according to the present invention;
FIG. 13 is a schematic view of an installation state of step S600 during an assembly process of a novel Luban lock structure according to the present invention;
FIG. 14 is a schematic view of an installation state of step S700 during an assembly process of a novel Luban lock structure according to the present invention;
FIG. 15 is a schematic structural view of an unused state of a novel Luban lock structure according to the present invention;
FIG. 16 is a schematic structural view of a state where a slider is reset and engaged with a hook in a novel Luban lock structure according to the present invention;
FIG. 17 is a schematic structural view of a drug delivery booster device module in an autoinjector according to the present invention (where B is the novel Luban lock structure);
FIG. 18 is a schematic structural view of a drug delivery protection device module in an autoinjector according to the present invention;
FIG. 19 is an overall schematic structural view of an autoinjector according to the present invention;
FIG. 20 is a schematic structural view of a prompt unit of an autoinjector according to the present invention;
FIG. 21 is a schematic structural view of a sound-generating structure in a prompt unit of an autoinjector according to the present invention;
FIG. 22 is a schematic exploded structural view of a sound-generating structure in a prompt unit of an autoinjector according to the present invention;
FIG. 23 is a schematic structural view of an airbag structure of an autoinjector according to the present invention.

In the figures: 100: booster rod, 200: power source, 300: fixing block, 400: guide pin, 500: sound-emitting block, and 600: slider;
1: first boss, 2: upper inclined surface, 3: lower inclined surface, 4: positioning flat, 5: first cantilever, 6: recess inclined surface, 7: guiding inclined surface, 8: second cantilever, 9: hook inclined surface, 10: hook, 11: channel, 12: limiting hole, 13: stop plate, 14: second boss, 15: support arm, 16: limiting slot, 17: first through hole, 18: first side recess, 19: second side recess, 20: fixing seat, 21: second through hole, 22: receiving slot, 23: third side recess, and 24: elongated slot;
101: first housing, 102: snap slot, 103: support power source, 104: second housing, 105: drug chamber, 106: rubber plug, 107: needle, 108: protective cover, and 109: telescopic shroud; and
111: seat body, 112: lug, 113: arcuate slot, 114: mounting slot, 115: circular hole, 116: sound-generating piece, 117: circular cap, 118: bearing plate, 119: airbag box, 120: airbag, 121: spring, 122: air outlet, 123: buffer cylinder, 124: pressure sensor, and 125: support plate.

### SPECIFIC IMPLEMENTATIONS

The following further describes the present invention in detail with reference to the drawings and embodiments, such that those skilled in the art can implement the present invention with reference to the description.

It should be understood that the terms, such as "have", "comprise", and "include" used herein, do not exclude the presence or addition of one or more other elements or a combination thereof.

### Embodiment 1:

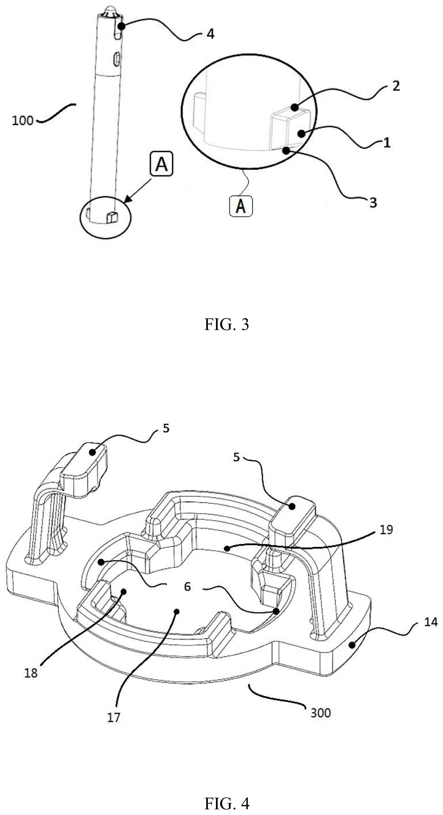

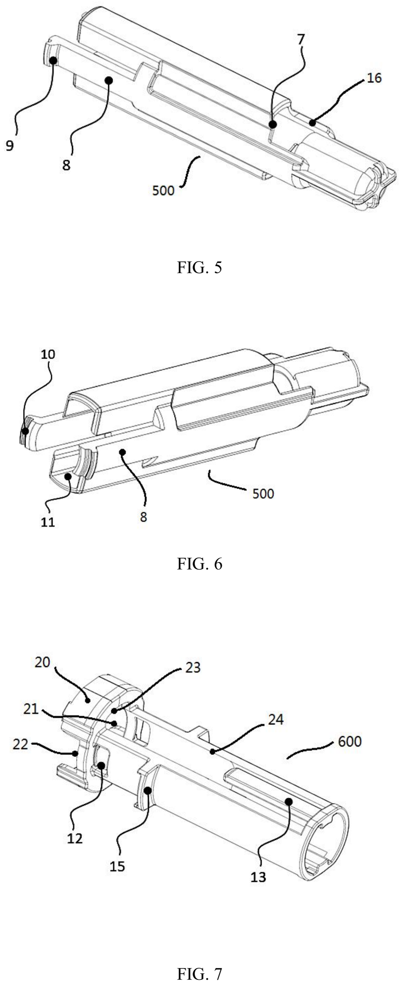

As shown in FIGS. 2-16, the present invention provides a novel Luban lock structure, including:
booster rod 100, power source 200, fixing block 300, guide pin 400, sound-emitting block 500, and slider 600, wherein the slider 600 is slidably coupled to an outer side of the sound-emitting block 500; the booster rod 100 is engaged with an inner side of the sound-emitting block 500 via first boss 1; the slider 600 is provided with a stop plate 13 configured to limit a position of the first boss 1; the fixing block 300 is snap-fitted between a top end of the sound-emitting block 500 and a top end of the slider 600; the guide pin 400 is connected to an inner bottom end of the sound-emitting block 500; the power source 200 is sleeved over the guide pin 400; and two ends of the power source 200 are respectively connected to an end of the guide pin 400 and an end of a cavity of the booster rod 100.

In the aforementioned Luban lock structure, the sound-emitting block 500 is configured as a hollow structure with an open top end, including:
limiting slots 16, wherein two limiting slots 16 are symmetrically formed at a lower part of the sound-emitting block 500 and extend through a side wall thereof;
channels 11, wherein two channels 11 are symmetrically connected to an outer side of the sound-emitting block 500 and extend along a length direction of the sound-emitting block 500; the channels 11 are in communication with the limiting slots 16, and a width of the limiting slots 16 is greater than a width of the channels 11; guiding inclined surface 7 is formed on a lower end surface where each channel 11 is connected to the corresponding limiting slot 16; and a height of the guiding inclined surface 7 gradually increases from an inner side to an outer side of the channel 11;
second cantilevers 8, wherein two second cantilevers 8 are symmetrically connected to an upper part of the sound-emitting block 500; the second cantilevers 8 and the channels 11 are arranged in a staggered manner, with a gap provided between them; and
hooks 10, wherein each hook 10 is connected to a top end of the second cantilever 8 and bent outward; and a bottom surface of each hook 10 is provided with hook inclined surface 9.

In the aforementioned Luban lock structure, the booster rod 100 is configured as a hollow structure with an open bottom end, including:
first bosses 1, wherein two first bosses 1 are symmetrically provided at both sides of a bottom end of the booster rod 100; an upper surface of each first boss 1 is provided with upper inclined surface 2 configured to be engaged with the guiding inclined surface 7 of the sound-emitting block 500; and a lower surface of each first boss 1 is provided with lower inclined surface 3; and
positioning flats 4, wherein two positioning flats 4 are symmetrically connected to a top end of the booster rod 100.

In the aforementioned Luban lock structure, the power source 200 is configured as an elastic reset element.

In the aforementioned Luban lock structure, the fixing block 300 includes:
first through hole 17 extending through a center of the fixing block 300;
second bosses 14, wherein two second bosses 14 are symmetrically connected to both sides of the fixing block 300;
first cantilevers 5, wherein each first cantilever 5 is connected to a top end of the second boss 14; and a top end of each first cantilever 5 is bent inward;
first side recesses 18, wherein two first side recesses 18 are symmetrically disposed in the first through hole 17 and correspond to positions of the first cantilevers 5; and recess inclined surface 6 is provided on a top surface of each first side recess 18, which is configured to be in contact with the hook inclined surface 9; and
second side recesses 19, wherein two second side recesses 19 are symmetrically disposed in the first through hole 17 and arranged in a staggered manner with the first side recesses 18; and a distance from each second side recess 19 to the center of the fixing block 300 is less than a distance from an outer side surface of the channel 11 to a center of the sound-emitting block 500.

In the aforementioned Luban lock structure, the slider 600 is configured as a hollow structure with two open ends, including:
fixing seat 20, wherein the fixing seat 20 is connected to a top end of the slider 600, and having a second through hole 21 formed through a center of the fixing seat 20; the fixing block 300 is mounted within the fixing seat 20; receiving slots 22 are symmetrically formed on both sides of the fixing seat 20; and the second boss 14 is mounted therein;
third side recesses 23, wherein two third side recesses 23 are symmetrically formed on both sides of the second through hole 21; the third side recesses 23 are arranged in a staggered manner with the receiving slots 22; a distance from each third side recess 23 to a center of the slider 600 is greater than the distance from the outer side surface of the channel to the center of the sound-emitting block 500; and the third side recesses 23 are configured to block the sound-emitting block 500;
limiting holes 12, wherein two limiting holes 12 are symmetrically formed in a side wall of the slider 600; and positions of the limiting holes 12 correspond to those of the receiving slots 22;
elongated slots 24, wherein two elongated slots 24 are symmetrically formed in both sides of the slider 600 and extend along a length direction of the slider 600; the elongated slots 24 are arranged in a staggered manner with the limiting holes 12 and communicate with the third side recesses 23; and the channel 11 is slidable into the corresponding elongated slot 24 along the third side recesses 23;
stop plates 13, wherein each stop plate 13 is connected to a bottom end of the elongated slot 24 and extends along the length direction of the slider 600; and the stop plate 13 is mounted in the channel 11; and
support arms 15, wherein two support arms 15 are symmetrically connected to an outer wall of the slider 600; and the support arms 15 are deposed below the limiting holes 12.

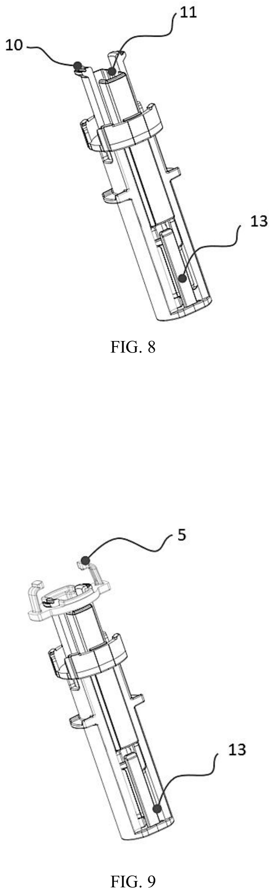

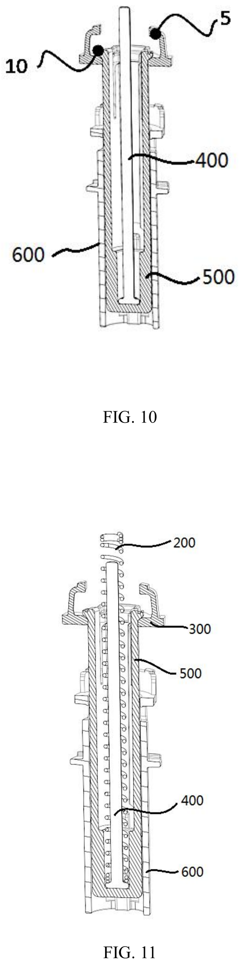

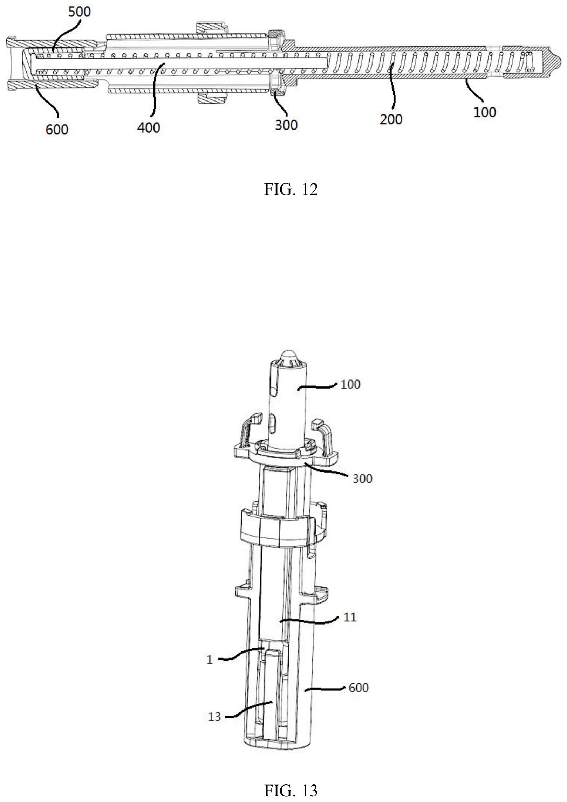

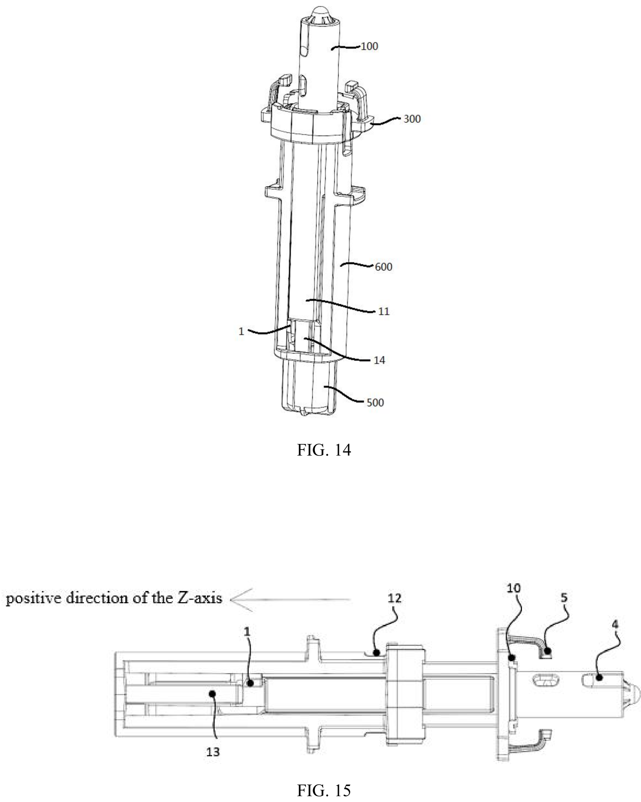

In this embodiment, a specific assembly process of the Luban lock structure is as follows:
step S100, inserting the sound-emitting block 500 into the slider 600, with an installation state as shown in FIG. 8;
wherein the channel 11 of the slider 600 is aligned with the third side recess 23, and as the sound-emitting block 500 is inserted, the channel 11 slides into the third side recess 23 and the elongated slot 24, at which time the stop plate 13 is not in contact with the channel 11;
step S200, fitting the fixing block 300 over the sound-emitting block 500, with an installation state as shown in FIG. 9;
wherein squeezing the two second cantilevers 8 to make the hooks 10 move closer to each other, while they pass through the first through hole 17 of the fixing block 300, and then releasing the two second cantilevers 8 so that the hook inclined surfaces 9 below the hooks 10 contact and snap-fit with the recess inclined surfaces 6 of the fixing block 300;
step S300, inserting the guide pin 400 into the sound-emitting block 500, with an installation state as shown in FIG. 10;
wherein installing a head of the guide pin 400 at an inner wall bottom end of the sound-emitting block 500 such that the guide pin 400 does not shake, with a top end of the guide pin 400 extending out of the sound-emitting block 500;
step S400, fitting the power source 200 over the guide pin 400 via a center thereof, with an installation state as shown in FIG. 11;
wherein the power source 200 is configured as an elastic reset element such as a spring or U-poly glue, and in this embodiment, a spring is used; fitting the power source 200 over the guide pin 400, one end of the power source 200 is connected to the head of the guide pin 400, the other end extends out of the sound-emitting block 500, and a length of the power source 200 in a free state is greater than a length of the guide pin 400;
step S500, fitting the booster rod 100 over the power source 200, with an installation state as shown in FIG. 12;
step S600, pressing the booster rod 100 to pass through the sound-emitting block 500, then rotating the booster rod 100 to engage the first bosses 1 of the booster rod 100 with the guiding inclined surfaces 7 of the sound-emitting block 500, with an installation state as shown in FIG. 13;
wherein pressing the booster rod 100 so that the power source 200 abuts against a top end of an inner wall of the booster rod 100, the first bosses 1 of the booster rod 100 align with the channels 11, as the booster rod 100 is pressed, the power source 200 is pre-compressed, the first bosses 1 slide along the channels 11 until entering the limiting slots 16, then rotating the booster rod 100 so that the first bosses 1 disengage from the channels 11 and are engaged with the guiding inclined surfaces 7; and
step S700, pushing the slider 600 upward to insert the two stop plates 13 of the slider 600 into the corresponding channels 11 of the sound-emitting block 500, thereby completing the assembly, with an installation state as shown in FIG. 14;
wherein the stop plates 13 of the slider 600 are aligned with the channels 11 of the sound-emitting block 500, pushing the slider 600 to move upward so that the stop plates 13 are inserted into the channels 11, the first bosses 1 abut against sides of the stop plates 13, and the channels 11 limit the stop plates 13 so that the first bosses 1 are prevented from moving; at this time, the second bosses 14 are snap-fitted into the receiving slots 22 of the slider 600, thus completing the assembly of the Luban lock structure.

The working principle and beneficial effects of the above technical solution are as follows:
In the novel Luban lock structure provided by the present invention, a central axis of the Luban lock along the length direction is set as a Z-axis, an installation direction of the slider 600 is a positive direction of the Z-axis, and an installation direction of the booster rod 100 is a negative direction of the Z-axis. When the Luban lock structure is used, the slider 600 is moved in the positive direction of the Z-axis until the stop plates 13 on the slider 600 completely exit the channels 11 of the sound-emitting block 500, at which time the first bosses 1 of the booster rod 100 lose limitation; under the action of the power source 200 and the guiding inclined surfaces 7 of the sound-emitting block 500, the booster rod 100 rotates along the guiding inclined surfaces 7 of the sound-emitting block 500; when the first bosses 1 of the booster rod 100 rotate into the channels 11 of the sound-emitting block 500, they start to move in the negative direction of the Z-axis; after the booster rod 100 completely moves out of the sound-emitting block 500, the power source 200 still maintains a certain force, which will push the sound-emitting block 500 to move in the positive direction of the Z-axis and collide with the subsequent components, thereby forming a sound. When the slider 600 is reset (moving in the negative direction of the Z-axis), the hook inclined surfaces 9 on the sound-emitting block 500 slide relative to the recess inclined surfaces 6, the hooks 10 are compressed into the slider 600, and as they slide, the hooks 10 snap-fit into the limiting holes 12 on the side of the slider 600, thereby forming protection.

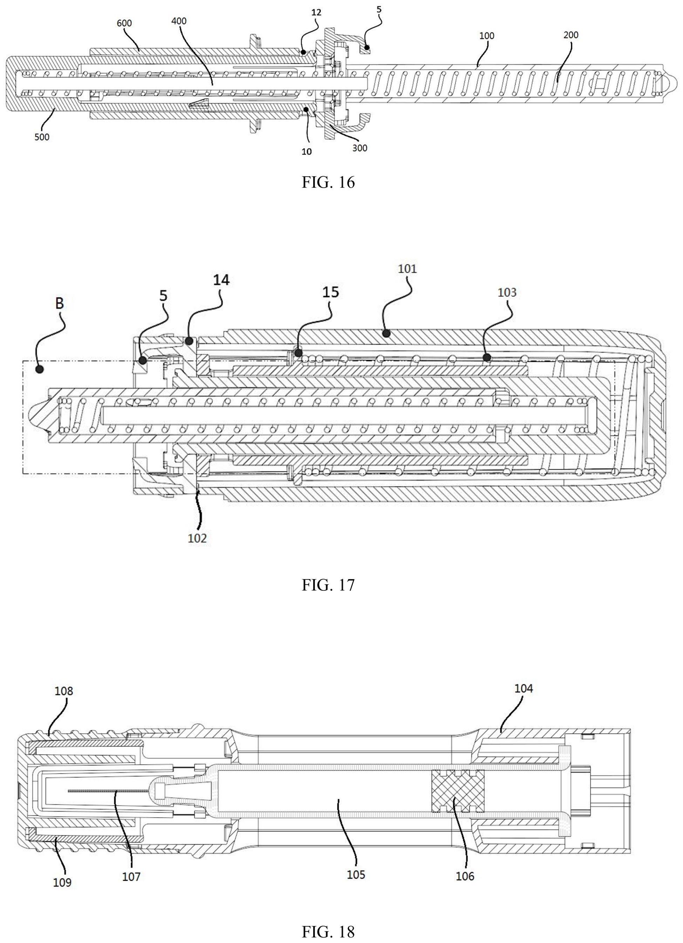

As shown in FIGS. 17-19, the present invention further provides a method for applying a novel Luban lock structure to an autoinjector. By adopting the novel Luban lock structure, the Luban lock structure is installed in an autoinjector to form a drug delivery booster device module. The drug delivery booster device module includes:
a first housing 101, wherein the first housing 101 is sleeved over an outer side of the novel Luban lock structure and having one end provided with an opening; the booster rod 100 extends out of the first housing 101; a side wall of the first housing 101 is symmetrically provided with snap slots 102; an edge of the second boss 14 of the fixing block 300 is snap-fitted within the snap slot 102; and an end of the sound-emitting block 500 is spaced apart from an end of an inner wall of the first housing 101; and
a support power source 103, wherein the support power source 103 is sleeved over on an outer side of the slider 600; two ends of the support power source 103 are respectively connected to the end of the inner wall of the first housing 101 and the support arm 15; the support power source 103 is configured as an elastic reset element such as a spring or U-poly glue; and in this embodiment, a spring is adopted.

The autoinjector further includes a drug delivery protection device module, and the drug delivery protection device module includes:
a second housing 104 having one end connected to an open end of the first housing 101, and the other end provided with an opening;
a drug chamber 105, wherein the drug chamber 105 is installed at a center of the second housing 104; a drug liquid is accommodated within the drug chamber 105 and is blocked by a rubber plug 106; the first cantilever 5 is snap-fitted with one end of the drug chamber 105; the booster rod 100 extends into the drug chamber 105 and is adjacent to the rubber plug 106; and the other end of the drug chamber 105 is connected to a needle 107;
a protective cover 108 detachably connected to an open end of the second housing 104 and fitted over an outer side of the needle 107; and
a telescopic shroud 109, wherein the telescopic shroud 109 is telescopically connected to the open end of the second housing 104; and the telescopic shroud 109 is connected to the slider 600 through a structural member.

The working principle and beneficial effects of the above technical solution are as follows:
The Luban lock structure plays a role of boosting in the autoinjector. When the autoinjector is assembled:
First, combined with the first housing 101 and the support power source 103, the Luban lock structure is installed into the first housing 101, the second bosses 14 of the fixing block 300 are snap-fitted within the snap slots 102 on the first housing 101, and simultaneously the support power source 103 is supported on the support arms 15 of the slider 600, forming the drug delivery booster device module.

Then, at a pharmaceutical factory, the drug delivery booster device module is connected to the drug delivery protection device module in which the drug chamber is pre-installed.

When the autoinjector is used, the protective cover 108 is first removed, then the telescopic shroud 109 is aligned with a site for drug delivery, and the entire device is pressed to cause the needle 107 to pierce the skin. At this time, the telescopic shroud 109 retracts inwardly and simultaneously drives the slider 600 to retreat. When the stop plates 13 of the slider 600 completely exit the channels 11 of the sound-emitting block 500, the first bosses 1 of the booster rod 100 lose limitation. Under the action of the power source 200 and the guiding inclined surfaces 7 of the sound-emitting block 500, the booster rod 100 rotates along the guiding inclined surfaces 7 of the sound-emitting block 500. When the first bosses 1 of the booster rod 100 rotate into the channels 11 of the sound-emitting block 500, they start to move in the negative direction of the Z-axis, thereby pushing the rubber plug 106 or other equivalents in the drug chamber 105 to squeeze the drug liquid out of the needle 107, achieving self-administration. When the drug delivery is completed, the booster rod 100 will completely disengage from the sound-emitting block 500. At this time, the power source 200 still maintains a certain force, which will push the sound-emitting block 500 to move backward (positive direction of the Z-axis ) and collide with the subsequent components, thereby forming a sound to remind that the drug delivery is completed. After the user completes the drug delivery and removes the device, the slider 600 is reset (moved in the negative direction of the Z-axis) under the action of the support power source 103, and the hooks 10 on the sound-emitting block 500 snap-fit with the limiting holes 12 on the side of the slider 600, thereby forming protection.

The novel Luban lock structure provided by the present invention achieves the following technical effects when applied to an autoinjector:
When the first bosses 1 of the booster rod 100 are engaged with the guiding inclined surfaces 7 of the sound-emitting block 500, and at the same time the stop plates 13 of the slider 600 enter the channels 11 of the sound-emitting block 500, the booster rod 100 is prevented from moving forward, which solves the problem of accidental triggering of the power unit of the current autoinjectors; the booster rod 100 is free of grooves, which increases its strength, reduces the risk of fracture during long-term storage, extends the service life of the autoinjector, lowers the requirements for structural manufacturing precision, and reduces production costs.

The novel Luban lock structure provided by the present invention belongs to a core structure in a single-use automatic injection device in the field of self-administration. The structure can also be applied to other self-administration products, such as emergency injection pens and microneedle drug delivery device.

### Embodiment 2:

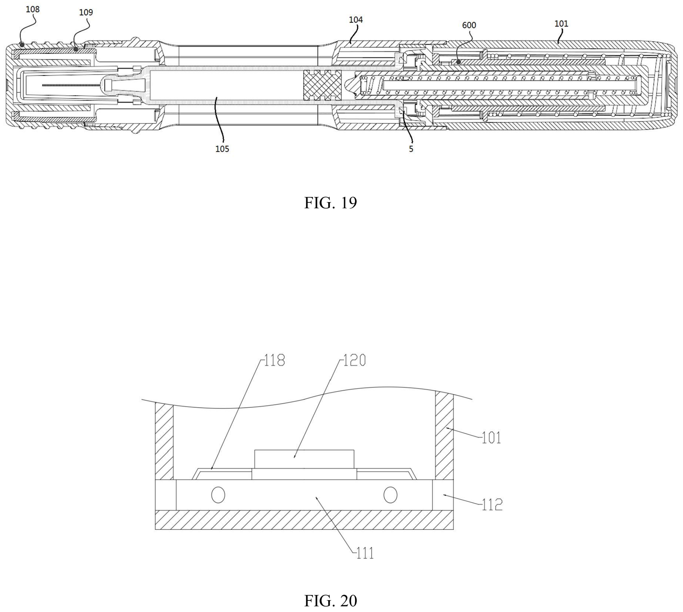

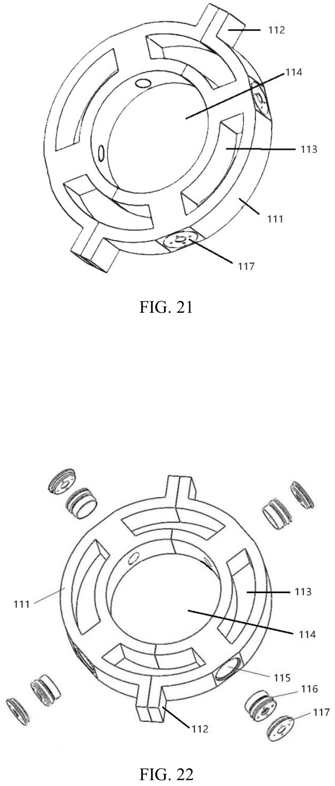

As shown in FIGS. 20-22, on the basis of Embodiment 1, a prompt unit is connected to an end of an inner wall of the first housing 101. The prompt unit includes a sound-generating structure and an airbag structure. The sound-emitting block 500 squeezes the airbag structure to supply air to the sound-generating structure. The sound-generating structure includes:
a seat body 111, wherein the seat body 111 is formed by joining two semi-circular plates together; a mounting slot 114 is formed in a center of a top end of the seat body 111; the mounting slot 114 is in communication with an air outlet end of the airbag structure; four circular holes 115 are uniformly formed in an outer ring end of the seat body 111; the circular holes 115 are connected to the mounting slot 114 through air channels; and an inner wall of each circular hole 115 is provided with threads;
lugs 112, wherein two lugs 112 are symmetrically connected to both sides of the seat body 111 and located on a joining surface of the two semi-circular plates; and the lugs 112 are snap-fitted into the snap slots on the side wall of the first housing 101;
arcuate slots 113, wherein four arcuate slots 113 are formed on the seat body 111 and uniformly arranged along a circumferential direction;
sound-generating pieces 116, wherein the sound-generating pieces 116 are fitted into the circular holes 115; an airflow channel is provided at a center of each sound-generating piece 116; and a reed is mounted in the airflow channel; and
circular caps 117, wherein the circular caps 117 are screwed onto the circular holes 115 and abut against the sound-generating pieces 116; and each circular cap 117 is provided with a through hole to allow airflow to pass through.

The working principle and beneficial effects of the above technical solution are as follows:
When the sound-generating structure is used, the seat body 111 adopts a split-type configuration and is snap-fitted to the first housing 101 through the lugs 112, which facilitates the assembly of the seat body 111. Under the squeezing action of the sound-emitting block 500, the airflow in the airbag structure enters the mounting slot 114, and then enters the circular holes 115 through the air channels. The airflow flows along the airflow channels of the sound-generating pieces 116 and drives the reeds to vibrate, causing the sound-generating pieces 116 to emit a sound to prompt the user that the injection is completed. The circular caps 117 are screwed onto the circular holes 115 to fix the sound-generating pieces 116. Through the above structural design, when the sound-emitting block 500 impacts the airbag structure, the squeezed airflow causes the sound-generating pieces 116 to emit a sound to prompt the user, avoiding situations where the sound cannot be effectively identified by relying solely on structural collision, thus enabling a more accurate determination of the time point of injection completion, and facilitating the use of the autoinjector.

### Embodiment 3:

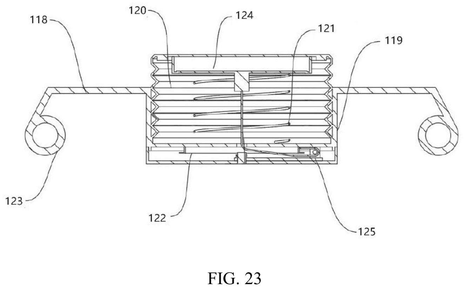

As shown in FIGS. 20 and 23, on the basis of Embodiment 2, the airbag structure includes:
a bearing plate 118, wherein the bearing plate 118 is provided above the seat body 111, and an outer side end of the bearing plate 118 is bent downward; an airbag box 119 is connected to a center of the bearing plate 118, and the airbag box 119 is connected within the mounting slot 114; and an air outlet 126 at a bottom end of the airbag box 119 is in communication with an interior of the mounting slot 114;
an airbag 120, wherein a bottom end of the airbag 120 is connected within the airbag box 119, with a top end of the airbag 120 protruding from the airbag box 119; and a spring 121 is connected between the top end and the bottom end of the airbag 120; and
a buffer cylinder 123 connected to a bottom end of the bearing plate 118 and slidably connected within the arcuate slots 113.

The working principle and beneficial effects of the above technical solution are as follows:
When the airbag structure is used, the sound-emitting block 500 impacts the airbag 120, squeezing the air in the airbag 120 to flow into the mounting slot 114 through the air outlet 126 at the bottom end of the airbag box; the spring 121 inside the airbag 120 exerts a buffering effect on the sound-emitting block 500; when the sound-emitting block 500 moves into contact with the bearing plate 118, it stops moving due to the blocking effect of the bearing plate 118, ensuring a consistent movement distance of the sound-emitting block 500 during use; at the same time, the sound-emitting block 500 impacts the bearing plate 118 and causes it to vibrate, and the vibration waves are transmitted to the buffer cylinder 123 through the bent end of the bearing plate 118; the cylindrical structure of the buffer cylinder 123 extends the transmission path of the vibration waves, thereby further dissipating the vibration waves; meanwhile, the buffer cylinder 123 vibrates along the inner walls of the arcuate slots 113 to achieve a vibration reduction effect. Through the above structural design, while ensuring a consistent movement distance of the sound-emitting block 500 during use and effective squeezing of the airbag 120, the impact generated by collision is reduced, and the vibration waves are effectively dissipated. This prevents the impact from the collision of the sound-emitting block 500 from being transmitted to the first housing 101 and then to the user's palm, reduces the risk of accidental slippage caused by palm impact, and improves the safety of the autoinjector during use.

### Embodiment 4:

As shown in FIG. 23, on the basis of Embodiment 3, the airbag structure further includes:
a pressure sensor 124 connected within a sensor box at the top end of the airbag 120, wherein a lead wire of the pressure sensor 124 is connected to a control panel of the autoinjector; and
a support plate 125 connected to the bottom end of the bearing plate 118 and configured as a sheet-like structure, wherein the support plate 125 serves to support and guide the lead wire of the pressure sensor 124.

The working principle and beneficial effects of the above technical solution are as follows:
The pressure sensor 124 is provided at the top end of the airbag 120 for detecting the pressure exerted on the airbag 120 by the sound-emitting block 500. When a detected pressure value is less than a preset value, it indicates that the pressure of the sound-emitting block 500 is insufficient, which indicates that the pressure of the power source 200 on the sound-emitting block 500 is insufficient, and the power source 200 cannot achieve a preset power effect. In this case, the autoinjector is unable to perform effective injection, and the control panel displays fault information to prompt the user to replace or repair the autoinjector, which improves the reliability of the use of the autoinjector and avoids the problem of insufficient injection dosage caused by using the autoinjector when the power output is insufficient. The lead wires are guided and supported by the support plate 125 to avoid messy distribution of the lead wires.

In the description of the present invention, it should be understood that the orientation or positional relationships indicated by the terms such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial" and "circumferential" are based on the orientation or positional relationships shown in the drawings, which are merely for the convenience of describing the present invention and simplifying the description, rather than indicating or implying that the referred device or element must be constructed and operated with a specific orientation, and therefore cannot be construed as a limitation on the present invention.

In the present invention, unless otherwise clearly specified and defined, the terms such as "mounted", "connected", "coupled" and "fixed" should be understood in a broad sense. For example, they may refer to a fixed connection, a detachable connection, or integral formation; they may refer to a mechanical connection, an electrical connection, or mutual communication; they may refer to a direct connection, or an indirect connection through an intermediate medium, and they may also refer to the internal connectivity between two elements or the interaction relationship between two elements, unless otherwise clearly defined. For those skilled in the art, the specific meanings of the above terms in the present invention can be understood according to specific circumstances.

Although the embodiments of the present invention have been disclosed as above, they are not limited to applications listed in the specification and the embodiments. The present invention can be fully applied to various fields suitable for it. For those skilled in the art, additional modifications can be easily implemented. Therefore, without departing from the general concept defined by the claims and the scope of equivalents, the present invention is not limited to the specific details and the illustrative examples shown and described herein.

## Claims

1. A novel Luban lock structure, comprising:
a booster rod (100), a power source (200), a fixing block (300), a guide pin (400), a sound-emitting block (500), and a slider (600), wherein the slider (600) is slidably coupled to an outer side of the sound-emitting block (500); the booster rod (100) is engaged with an inner side of the sound-emitting block (500) via a first boss (1); the slider (600) is provided with a stop plate (13) configured to limit a position of the first boss (1); the fixing block (300) is snap-fitted between a top end of the sound-emitting block (500) and a top end of the slider (600); the guide pin (400) is connected to an inner bottom end of the sound-emitting block (500); the power source (200) is sleeved over the guide pin (400); and two ends of the power source (200) are respectively connected to an end of the guide pin (400) and an end of a cavity of the booster rod (100).

2. The novel Luban lock structure according to claim 1, wherein the sound-emitting block (500) is configured as a hollow structure with an open top end, comprising:
limiting slots (16), wherein two limiting slots (16) are symmetrically formed at a lower part of the sound-emitting block (500) and extend through a side wall thereof;
channels (11), wherein two channels (11) are symmetrically connected to an outer side of the sound-emitting block (500) and extend along a length direction of the sound-emitting block (500); the channels (11) are in communication with the limiting slots (16), and a width of the limiting slots (16) is greater than a width of the channels (11); a guiding inclined surface (7) is formed on a lower end surface where each channel (11) is connected to the corresponding limiting slot (16); and a height of the guiding inclined surface (7) gradually increases from an inner side to an outer side of the channel (11);
second cantilevers (8), wherein two second cantilevers (8) are symmetrically connected to an upper part of the sound-emitting block (500); the second cantilevers (8) and the channels (11) are arranged in a staggered manner, with a gap provided between them; and
hooks (10), wherein each hook (10) is connected to a top end of the second cantilever (8) and folded outward; and a bottom surface of each hook (10) is provided with a hook inclined surface (9).

3. The novel Luban lock structure according to claim 2, wherein the booster rod (100) is configured as a hollow structure with an open bottom end, comprising:
first bosses (1), wherein two first bosses (1) are symmetrically provided at both sides of a bottom end of the booster rod (100); an upper surface of each first boss (1) is provided with an upper inclined surface (2) configured to be engaged with the guiding inclined surface (7) of the sound-emitting block (500); and a lower surface of each first boss (1) is provided with a lower inclined surface (3); and
positioning flats (4), wherein two positioning flats (4) are symmetrically connected to a top end of the booster rod (100).

4. The novel Luban lock structure according to claim 1, wherein the power source (200) is configured as an elastic reset element.

5. The novel Luban lock structure according to claim 3, wherein the fixing block (300) comprises:
a first through hole (17) extending through a center of the fixing block (300);
second bosses (14), wherein two second bosses (14) are symmetrically connected to both sides of the fixing block (300);
first cantilevers (5), wherein each first cantilever (5) is connected to a top end of the second boss (14); and a top end of each first cantilever (5) is bent inward;
first side recesses (18), wherein two first side recesses (18) are symmetrically disposed in the first through hole (17) and correspond to positions of the first cantilevers (5); and a recess inclined surface (6) is provided on a top surface of each first side recess (18), which is configured to be in contact with the hook inclined surface (9); and
second side recesses (19), wherein two second side recesses (19) are symmetrically disposed in the first through hole (17) and arranged in a staggered manner with the first side recesses (18); and a distance from each second side recess (19) to the center of the fixing block (300) is less than a distance from an outer side surface of the channel (11) to a center of the sound-emitting block (500).

6. The novel Luban lock structure according to claim 5, wherein the slider (600) is configured as a hollow structure with two open ends, and the slider (600) comprises:
a fixing seat (20), wherein the fixing seat (20) is connected to a top end of the slider (600) and having a second through hole (21) formed through a center of the fixing seat (20); the fixing block (300) is mounted within the fixing seat (20); receiving slots (22) are symmetrically formed on both sides of the fixing seat (20); and the second boss (14) is mounted therein;
third side recesses (23), wherein two third side recesses (23) are symmetrically formed on both sides of the second through hole (21); the third side recesses (23) are arranged in a staggered manner with the receiving slots (22); and a distance from each third side recess (23) to a center of the slider (600) is greater than the distance from the outer side surface of the channel (11) to the center of the sound-emitting block (500); and
limiting holes (12), wherein two limiting holes (12) are symmetrically formed in a side wall of the slider (600); and positions of the limiting holes (12) correspond to those of the receiving slots (22).

7. The novel Luban lock structure according to claim 6, wherein the slider (600) further comprises:
elongated slots (24), wherein two elongated slots (24) are symmetrically formed in both sides of the slider (600) and extend along a length direction of the slider (600); the elongated slots (24) are arranged in a staggered manner with the limiting holes (12) and communicate with the third side recesses (23); and the channel (11) is slidable into the corresponding elongated slot (24) along the third side recesses (23); and
stop plates (13), wherein each stop plate (13) is connected to a bottom end of the elongated slot (24) and extends along the length direction of the slider (600); and the stop plate (13) is mounted in the channel (11); and
support arms (15), wherein two support arms (15) are symmetrically connected to an outer wall of the slider (600); and the support arms (15) are disposed below the limiting holes (12).

8. The novel Luban lock structure according to claim 1, wherein its specific assembly process comprises the following steps:
step S100, inserting the sound-emitting block (500) into the slider (600);
step S200, fitting the fixing block (300) over the sound-emitting block (500);
step S300, inserting the guide pin (400) into the sound-emitting block (500);
step S400, fitting the power source (200) over the guide pin (400) via a center thereof;
step S500, fitting the booster rod (100) over the power source (200);
step S600, pressing the booster rod (100) to pass through the sound-emitting block (500), then rotating the booster rod (100) to engage the first bosses (1) of the booster rod (100) with the guiding inclined surfaces (7) of the sound-emitting block (500); and
step S700, pushing the slider (600) upward to insert the two stop plates (13) of the slider (600) into the corresponding channels (11) of the sound-emitting block (500), thereby completing the assembly.

9. A method for applying a novel Luban lock structure to an autoinjector, employing the novel Luban lock structure according to claim 7, wherein the novel Luban lock structure is installed within the autoinjector to form a drug delivery booster device module, and the drug delivery booster device module comprises:
a first housing (101), wherein the first housing (101) is sleeved over an outer side of the novel Luban lock structure and having one end provided with an opening; the booster rod (100) extends out of the first housing (101); a side wall of the first housing (101) is symmetrically provided with snap slots (102); an edge of the second boss (14) of the fixing block (300) is snap-fitted within the snap slot (102); and an end of the sound-emitting block (500) is spaced apart from an end of an inner wall of the first housing (101); and
a support power source (103), wherein the support power source (103) is sleeved over an outer side of the slider (600); and two ends of the support power source (103) are respectively connected to the end of the inner wall of the first housing (101) and the support arm (15).

10. The method for applying a novel Luban lock structure to an autoinjector according to claim 9, wherein the autoinjector further comprises a drug delivery protection device module, and the drug delivery protection device module comprises:
a second housing (104) having one end connected to an open end of the first housing (101), and the other end provided with an opening;
a drug chamber (105), wherein the drug chamber (105) is installed at a center of the second housing (104); a drug liquid is accommodated within the drug chamber (105) and is blocked by a rubber plug (106); the first cantilever (5) is snap-fitted with one end of the drug chamber (105); the booster rod (100) extends into the drug chamber (105) and is adjacent to the rubber plug (106); and the other end of the drug chamber (105) is connected to a needle (107);
a protective cover (108) detachably connected to an open end of the second housing (104) and fitted over an outer side of the needle (107); and
a telescopic shroud (109), wherein the telescopic shroud (109) is telescopically connected to the open end of the second housing (104); and the telescopic shroud (109) is connected to the slider (600) via a structural member.
